(19) Europäisches Patentamt
European Patent Office
Office européen des brevets

(11) Veröffentlichungsnummer: **0 101 897**
**B1**

(12) # EUROPÄISCHE PATENTSCHRIFT

(45) Veröffentlichungstag der Patentschrift: **15.06.88**

(21) Anmeldenummer: **83107174.1**

(22) Anmeldetag: **22.07.83**

(51) Int. Cl.$^4$: **C 07 D 417/04,**
C 07 D 413/04, C 09 B 57/00,
C 09 B 23/10

(54) **Heterocyclische Verbindungen.**

(30) Priorität: **05.08.82 DE 3229301**

(43) Veröffentlichungstag der Anmeldung:
**07.03.84 Patentblatt 84/10**

(45) Bekanntmachung des Hinweises auf die Patenterteilung:
**15.06.88 Patentblatt 88/24**

(84) Benannte Vertragsstaaten:
**CH DE GB LI**

(56) Entgegenhaltungen:
**EP-A-0 021 304**
**EP-A-0 025 136**
**EP-A-0 027 529**
**GB-A-2 006 253**

(73) Patentinhaber: **BAYER AG**
**Konzernverwaltung RP Patentabteilung**
**D-5090 Leverkusen 1 Bayerwerk (DE)**

(72) Erfinder: **Seng, Florin, Dr.**
**Am Katterbach 46**
**D-5060 Bergisch-Gladbach 2 (DE)**
Erfinder: **Claussen, Uwe, Dr.**
**Carl-Rumpff-Strasse 29**
**D-5090 Leverkusen 1 (DE)**
Erfinder: **Beck, Gunther, Dr.**
**Am Mittelberg 19**
**D-5090 Leverkusen 1 (DE)**
Erfinder: **Sasse, Klaus, Dr.**
**Pützweg 13**
**D-5060 Bergisch-Gladbach 2 (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 101 897 B1

**Beschreibung**

Die Erfindung betrifft heterocyclische Verbindungen der Formel

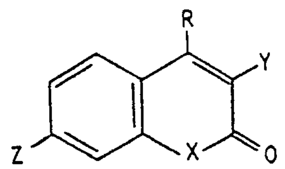

I

Verfahren zu ihrer Herstellung sowie ihre Verwendung.

Aus den Literaturstellen EP—A1—21304, EP—A1—25136, EP—A1—27529 und GB—A—2 006 253 sind 7-substituierte 4-Cycanurcumarine bekannt, die in 3-Stellung durch u.a. heterocyclische Reste substitutiert sein können; ein Hinweis auf eine Substitution in 3-Stellung durch die ausgewählten Reste

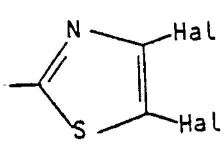

und

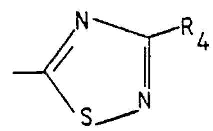

wobei Hal Halogen und $R_4$ Wasserstoff, $C_1$—$C_6$-Alkyl oder Phenyl bedeutet, ist den genannten Literaturstellen nicht zu entnehmen.

Aus Chemical Abstracts 89 (1978), Referat 179907p ist eine Verbindung der Formel

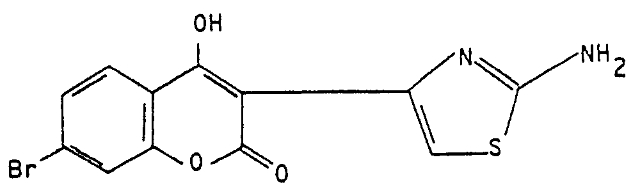

bekannt.

In Formel I bezeichnen:

R Cl, Br, CN oder OH;

X O, S oder —$NR_1$, wobei

$R_1$ für Wasserstoff, $C_1$—$C_6$-Alkyl, $C_3$—$C_7$-Cycloalkyl, Phenyl, Naphthyl, Phenyl-$C_1$—$C_4$-alkyl, Naphthyl-$C_1$—$C_4$-alkyl, ($C_1$—$C_6$-Alkyl)-carbonyl, Benzoyl steht und die für $R_1$ genannten $C_1$—$C_6$-Alkyl- und ($C_1$—$C_6$-Alkyl)-carbonylreste durch —OH, —CN, $C_1$—$C_6$-Alkoxy, Phenoxy, Halogen, Amino, Carbamoyl, Sulfamoyl, ($C_1$—$C_6$-Alkyl)-carbonyloxy, ($C_1$—$C_6$-Alkoxy)-carbonyl, der $C_3$—$C_7$-Cycloalkylrest durch Halogen $C_1$—$C_6$-Alkyl, —OH, —CN, Phenyl, die Phenyl-, Naphthyl-, Benzoylreste sowie die Phenyl-$C_1$—$C_4$-alkyl und Naphthyl-$C_1$—$C_4$-alkylreste im Arylteil durch Halogen, $C_1$—$C_6$-Alkyl, $C_1$—$C_6$-Alkoxy, —CN, —OH substituiert sein können;

Y für einen 5- bis 7-gliedrigen heterocyclischen Rest, der 1, 2 oder 3 Atome aus der Reihe O, N, S enthalten kann und der anelliert sein kann, wobei die für Y genannten Reste durch Halogen, $C_1$—$C_6$-Alkyl, $C_1$—$C_6$-Alkoxy, $C_1$—$C_6$-Alkylthio, —$NO_2$, ($C_1$—$C_6$-Alkoxy)-carbonyl, Carbamoyl, Amino, Sulfamoyl substituiert sein können;

Z Cl, Br, —CN, $R_2$—T- oder $R'_2$ bedeutet, wobei

$R_2$ für Wasserstoff, $C_1$—$C_6$-Alkyl, das durch —OH, —CN, $C_1$—$C_6$-Alkoxy, Phenoxy, Halogen, Amino, Carbamoyl, Sulfamoyl, ($C_1$—$C_6$-Alkyl)-carbonyloxy, ($C_1$—$C_6$-Alkoxy)-carbonyl substituiert sein kann, $C_3$—$C_7$-Cycloalkyl, das durch Halogen, $C_1$—$C_6$-Alkyl, —OH, —CN, Phenyl substituiert sein kann, Phenyl und Naphthyl bzw. Phenyl-$C_1$—$C_4$-alkyl und Naphthyl-$C_1$—$C_4$-alkyl, wobei die Arylreste durch Halogen, $C_1$—$C_6$-Alkyl, $C_1$—$C_6$-Alkoxy, —CN, —OH substituiert sein können, steht,

$R'_2$ für Phenyl, Naphthyl, Phenyl-$C_1$—$C_4$-alkyl, Naphthyl-$C_1$—$C_4$-alkyl, wobei die Arylreste, wie vorstehend im Zusammenhang mit $R_2$ angegeben, substituiert sein können und

T für O, S, 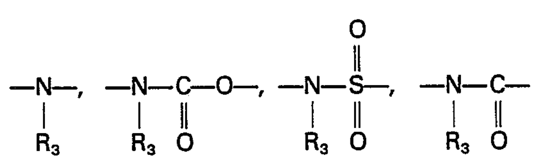

steht

$R_3$ die gleichen Bedeutungen wie $R_2$ hat, und wobei bei Vorliegen des Strukturmerkmals

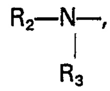

$R_2$ und $R_3$ zusammen mit dem N-Atom einen 5- bis 7-gliedrigen Heterocyclus bilden können, mit der Maßgabe, daß

a) für den Fall, daß R —CN bezeichnet, und X für

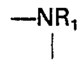

steht, Z nicht $R_2$—O— bedeutet und

b) für den Fall, daß R —CN bezeichnet und X für O steht,

Y 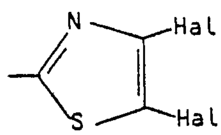 oder 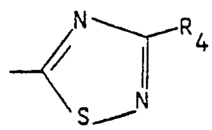

bedeutet, wobei Hal Halogen, vorzugsweise Chlor, und Brom und $R_4$ Wasserstoff, $C_1$—$C_6$-Alkyl oder Phenyl bezeichnen und

c) von diesem Anspruch die Verbindung der Formel

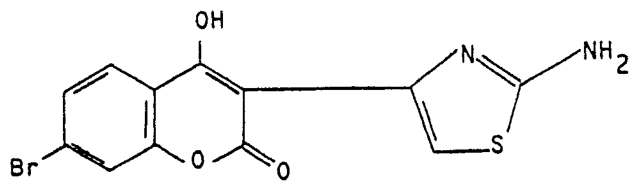

ausgenommen ist.

R bezeichnet bevorzugt Cl, CN oder OH.

X steht vorzugsweise für O oder —N—$R'_1$, wobei

$R'_1$ $C_3$—$C_7$-Cycloalkyl, Phenyl, Naphthyl, Phenyl-$C_1$—$C_4$-alkyl, Naphthyl-$C_1$—$C_4$-alkyl und insbesondere $C_1$—$C_5$-Alkyl bezeichnet.

Z steht bevorzugt für Di-($C_1$—$C_6$-alkyl)-amino oder $C_1$—$C_6$-Alkoxy.

$C_1$—$C_6$-Alkyl ($R_1$, $R_2$, $R_3$, $R'_1$) bezeichnet vorzugsweise Methyl, Ethyl, i-Propyl, n-Butyl, t-Butyl. Als Substituenten für $C_1$—$C_6$-Alkyl seien beispielhaft Chlor oder Brom genannt.

$C_3$—$C_7$-Cycloalkyl ($R_1$, $R_2$, $R_3$, $R'_1$) steht bevorzugt für Cyclopentyl und Cyclohexyl. Als Substituenten für Cycloalkyl seien beispielhaft Chlor und Brom genannt;

Als Substituenten für Phenyl und Naphthyl ($R_1$, $R_2$, $R_3$, $R'_1$) kommen beispielsweise Chlor und Brom in Betracht.

Phenyl-$C_1$—$C_4$-alkyl ($R_1$, $R_2$, $R_3$, $R'_1$) steht beispielsweise für Benzyl oder Phenethyl. Die Phenyl-$C_1$—$C_4$-alkyl- und Naphthyl-$C_1$—$C_4$-alkyl-Reste ($R_1$, $R_2$, $R_3$, $R'_1$) können im Arylrest z.B. durch Chlor oder Brom substituiert sein.

Als Substituenten der heterocyclischen Reste Y kommen beispielsweise Chlor oder Brom in Betracht.

Besonders bevorzugt steht Y für einen Rest aus der Thiazol-, Oxazol-, 1,3,4-Oxadiazol-, 1,3,4-Thiadiazol-, 1,2,4-Thiadiazol-, Benzthiazol- Benzoxazol-, imidazol-, 1,2,4-Oxadiazol-, Chinazolon-, Benzimidazol-, Pyridin-, Chinolin- oder Pyrimidin-Reihe, z.B. einen Benzthiazolyl(2)-, 5-Chlorbenzthiazolyl(2)-, 5-Methylbenzthiazolyl(2)-, 4,5-Dichlorthiazolyl(2)-, Benzoxazolyl(2)-, 5-Chlorbenzoxazolyl(2)- oder 5-Methylbenzoxazolyl(2)-Rest oder einen Rest der Formeln

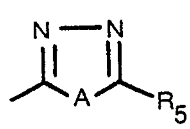 und 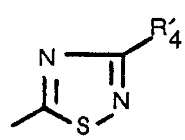

in denen

A für O oder S und

$R'_4$, $R_5$ für Wasserstoff oder $C_1$—$C_6$-Alkyl, insbesondere Methyl stehen.

Für den Fall, daß für Z das Strukturmerkmal

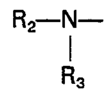

vorliegt, können $R_2$ und $R_3$ zusammen unter Einschluß des N-Atoms z.B. einen Piperidin-, Pyrrolidin-, Morpholin- oder Piperazin-Ring bilden.

Von besonderem Interesse im Rahmen der vorliegenden Erfindung sind Verbindungen der Formel I, bei denen

Y einen Rest

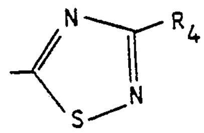

bezeichnet, insbesondere solche, bei denen zusätzlich R für CN steht.

Bevorzugt sind weiterhin Verbindungen der Formel

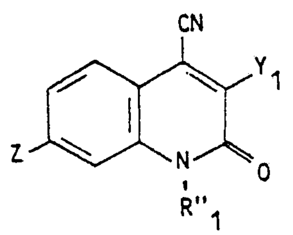

II

in der

$R''_1$ $C_1$—$C_6$-Alkyl oder Phenyl und

$Y_1$ einen gegebenenfalls durch Chlor, Brom oder $C_1$—$C_4$-Alkyl insbesondere Methyl substituierten Benzthiazolyl(2)-, Benzoxazolyl(2)-, Thiazolyl(2)-, 1,2,4-Thiazolyl(5)-, 1,3,4-Thiadiazolyl(2)-, oder 1,3,4-Oxadiazolyl(2)-Rest bezeichnen und

Z die zu Formel I angegebenen Bedeutungen hat, jedoch nicht $R_2$—O— bezeichnen kann.

Weiterhin bevorzugt sind Verbindungen der Formel

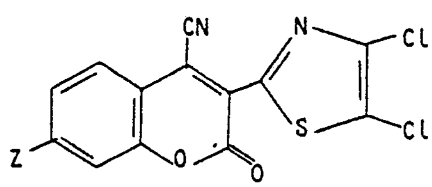

III

sowie Verbindungen der Formel

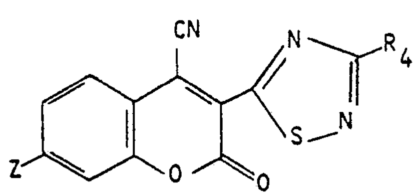

IV

Die Herstellung der Verbindungen der Formel I erfolgt beispielsweise in der Weise, daß man Verbindungen der Formel

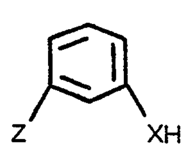

V

mit Verbindungen der Formel

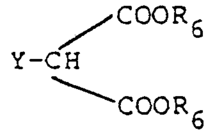

(VI)

in der

$R_6$ beispielsweise für einen $C_1$—$C_4$-Alkylrest steht, in An- oder Abwesenheit eines inerten organischen Verdünnungsmittels bei erhöhter Temperatur umsetzt.

Für diese Umsetzung geeignete inerte organische Verdünnungsmittel sind z.B. hochsiedende chlorierte Kohlenwasserstoffe wie z.B. o-Dichlorbenzol oder hochsiedende Ether wie z.B. Diphenylether.

Die Reaktion wird im Temperaturbereich von 100 bis 200°C, vorzugsweise bei 140 bis 180°C, durchgeführt.

Verwendet man als Ausgangsstoff der Formel V 3-Diethylaminophenol und als Ausgangsstoff der Formel VI Benzthiazolylmalonsäure-diethylester, so kann der Verlauf der Reaktion anhand folgender Formelgleichung wiedergegeben werden:

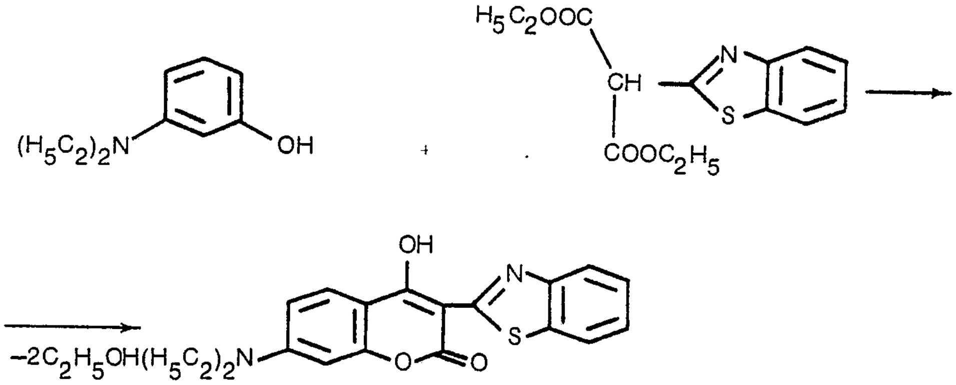


Die Verbindungen der Formel VI werden erhalten, wenn man die Verbindungen der allgemeinen Formel VII

Y—Cl VII

mit Malonestern VIII

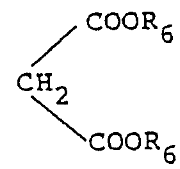

VIII

in einem inerten organischen Verdünnungsmittel in Gegenwart eines basischen Kondensationsmittels umsetzt.

Als Verdünnungsmittel kommen (cyclische) Ether, z.B. Dioxan, Tetrahydrofuran, aliphatische Ketone, z.B. Aceton, aromatische Kohlenwasserstoffe, z.B. Benzol, Toluol oder Xylole, außerdem Dimethylformamid, Dimethylsulfoxid oder Hexamethylphosphorsäuretriamid infrage.

Als basische Kondensationsmittel werden Alkalimetallalkoholate wie z.B. Natriummethylat oder Kalium-tert.-butylat, Alkalimetallhydride wie z.B. Natriumhydrid oder Alkalihydroxide wie z.B. Kaliumhydroxid oder Natriumhydroxid verwendet.

Die Reaktion wird im Temperaturbereich von 0 bis 150°C, insbesondere bei 20 bis 120°C durchgeführt.

Pro Mol VII werden 1 bis 1,2 Mol Malonester VIII und 1 bis 3 Mol basisches Kondensationsmittel zugesetzt.

Die Verbindungen der Formel VII sind bekannt (siehe z.B. DE—OS 2 213 865; Indian J. Chem. (1969), 7, 760).

Die wie vorstehend beschrieben erhältlichen Verbindungen der Formel

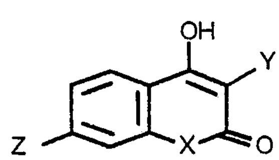

(IX)

lassen sich mit Chlorierungsmitteln in An- oder Abwesenheit von inerten organischen Verdünnungsmitteln zu Verbindungen der Formel

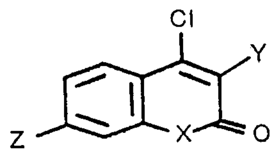

(X)

umsetzen.

Als Chlorierungsmittel kommen insbesondere Phosphoroxychlorid, Phosphorpentachlorid, Thionylchlorid, Oxalylchlorid oder Phosgen infrage.

Für die Umsetzung geeignete inerte organische Verdünnungsmittel sind vorzugsweise chlorierte Kohlenwasserstoffe wie Chloroform, Tetrachlorkohlenstoff, Dichlorethan, Chlorbenzol und o-Dichlorbenzol.

Die Umsetzung wird im Temperaturbereich von 50 bis 140°C, vorzugsweise bei 80 bis 120°C, durchgeführt.

Zur Reaktionsdurchführung suspendiert man die zu chlorierenden Verbindung IX in einem geeigneten Verdünnungsmittel und versetzt sie bei der Reaktionstemperatur mit dem Chlorierungsmittel. Verwendet man als Chlorierungsmittel Phosphorpentachlorid oder Phosphoroxychlorid, so hat sich die Verwendung von Phosphoroxychlorid als Reaktionsmedium bewährt. Nach beendeter Chlorierung wird das Lösungsmittel abdestilliert, wobei die chlorierte Verbindung zurückbleibt. In manchen Fällen hat es sich als zweckmäßig erwiesen, die Chlorierung in Gegenwart von katalytischen Mengen Dimethylformamid durchzuführen.

In analoger Weise erhält man bei Einsatz von Bromierungsmitteln Verbindungen der Formel I, in der R für Br steht.

Ausgehend von Verbindungen der Formel X eröffnet sich ein Verfahren zur Herstellung von Verbindungen der Formel

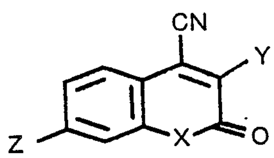

(XI)

in der

X, Y und Z die zu Formel I angegebenen Bedeutungen haben.

Dieses Verfahren ist dadurch gekennzeichnet, daß man Verbindungen der Formel X in einem organischen Lösungs- oder Verdünnungsmittel, vorzugsweise in einem polaren organischen Lösungsmittel, mit einem Metallcyanid umsetzt.

Für die Umsetzung geeignete polare organische Lösungsmittel sind z.B. Formamide, z.B. Formamid, Dimethylformamid und N-Methylpyrrolidon, Nitrile, z.B. Acetonitril oder Dimethylsulfoxid.

Die Umsetzung wird vorzugsweise im Temperaturbereich von 50 bis 140°C, vorzugsweise bei 70 bis 110°C durchgeführt.

Als Metallcyanide kommen z.B. Alkalimetallcyanide wie Natriumcyanid oder Kaliumcyanid sowie Schwermetallcyanide wie Kupfer(I)-cyanid, Quecksilber(II)-cyanid oder Zink(II)-cyanid infrage.

Zur Durchführung der Reaktion wird die Verbindung der allgemeinen Formel X in einem geeigneten organischen Lösungs- oder Verdünnungsmittel suspendiert oder gelöst, mit einem Metallcyanid versetzt und bis zum Ende der Reaktion bei einer entsprechenden Reaktionstemperatur gerührt.

Nach beendeter Umsetzung wird das ausgefallene schwerlösliche Umsetzungsprodukt abgesaugt und in Wasser ausgerührt, um die anorganischen Anteile zu entfernen. In vielen Fällen hat es sich als zweckmäßig erwiesen, dem Ansatz eine katalytische menge (bis zu 10 Mol %) Kaliumjodid zuzusetzen.

Die Verbindungen der Formel I eignen sich zum Färben oder Bedrucken von synthetischen und halbsynthetischen Materialien insbesondere von Textilmaterial z.B. aus Polyamiden, Triacetat, und insbesondere Polyestern, sowie zum Färben von Kunststoffen in der Masse und für den Transferdruck. Sie liefern brillante, farbstarke Färbungen oder Drucke mit guten Echtheiten.

Weiterhin eignen sich die Verbindungen der Formel I, insbesondere solche, bei denen R für Cyan steht als Lichtumwandler in Lichtsammelsystemen (s. z.B. DE—OS 2 934 541).

Die Lichtsammelsysteme, bei denen es sich um Systeme der genannten Farbstoffe in Matrizen geeigneter Geometrie, d.h. optischen Systemen, bei denen das Verhältnis von Emissions- zu Absorptionsflächen 1:100 bis 1:1000 betragt kann, handelt, sind geeignet, einfallende diffuse elektromagnetische Strahlung zu absorbieren und in einem gegenüber der Umgebung optisch dichteren Medium nahezu verlustlos zu emittieren, wobei der Hauptteil des Emissionslichtes total reflektiert im Medium verbleibt.

Nur der Anteil des emittierten Lichtes, dessen Emissionsbande weitgehend frei von Absorption ist, ist

für den erfindungsgemäßen Zweck nutzbar.

Deshalb ist es zweckmäßig, die Farbstoffe der Formel (I) von Einsatz in die Lichtsammelsysteme besonders zu reinigen. Die Erfindung betrifft demnach bevorzugt Lichtsammelsysteme, die dadurch gekennzeichnet sind, daß sie einen Farbstoff der Formel I enthalten, der in einer 0,05 %igen Lösung, in einer Schichtdicke von 10 cm einen Anstieg der Transmission von 0 auf >90% bei einer Änderung der Wellenlänge von 25—100 nm, vorzugsweise 25—45 nm, aufweist.

Die Reinigung der Farbstoffe kann in günstigen Fällen durch mehrfache sorgfältige Kristallisation erfolgen. Zumeist ist eine säulenchromatographische Trennung an festen Trägern wie $Al_2O_3$ oder $SiO_2$ erforderlich. Besonders schonend erhält man hohe Reinheitsgrade verteilungschromatographisch, z.B. durch Steady-State-(O'Keefe)- oder Gegenstromverteilung nach Craig.

Die neuen Lichtsammelsysteme können z.B. in Verbindung mit Solarzellen zur Nutzbarmachung der Sonnenenergie und in Szintillatoren bekannter Art [s. z.B. J. B. Birks. The Theory and Practice of Scintillation Counting (Pergamon Press, London 1964); J. Opt. Am. *39*, 912 (1949); J. Appl. Phys. *40*, 3544 (1969); Nuclear Instruments a. Methods *87*, 111—123 (1970)] Verwendung finden. Darüber hinaus eignen sie sich in Verbindung mit elektronischen Steuerungen als Anzeigevorrichtungen mit sehr geringem Energieverbrauch. Darüber hinaus eignen sie sich ohne elektronische Bauteile für vielerlei Anzeige-, Hinweis- und Markierungszwecke, z.B. in passiven Anzeigeelementen, Hinweis- und Verkehrszeichen wie Ampeln.

Die erfindungsgemäßen Lichtsammelsysteme enthalten den Farbstoff in einer Flüssigkeit oder einem Festkörper gelöst, wobei je nach Einsatzgebiet des Lichtsammelsystems, verschiedenste geometrische Formen in Frage kommen. Geeignete feste Medien wie sie z.B. zum Sammeln von Licht in Verbindung mit Solarzellen und in passiven Anzeigeelementen eingesetzt werden sind z.B. lichtdurchlässige, optisch verwendbare Kunststoffe wie Kunststoffe aus Basis Polyvinylchlorid, Polyacrylat, Polymethylmethacrylat, Polycarbonat, Celluloseester, Styrol-Acrylnitril-Polymerisat. Des weiteren können die Lichtsammelsysteme den Farbstoff auch in einer Flüssigkeit z.B. Alkohol, Keton, Halogenkohlenwasserstoff, Ether gelöst enthalten. Gut geeignete Lösungsmittel sind z.B. Ethanol, Propanol, Methyl-ethyl-keton, Aceton, Cyclohexanon, Chloroform, Perchlorethylen, Glykolmonomethylether.

Die erfindungsgemäße Verwendung der Farbstoffe der Formel (I) ist im hohen Maße vorteilhaft, da sie neben einer guten Quantenausbeute und einem hohen Verstärkungsfaktor ausgezeichnet Lichtechtheit aufweisen und damit eine ökonomische Verwendbarkeit der neuen Lichtsammelsysteme gewährleisten.

Es muß als überraschend bezeichnet werden, daß die Farbstoffe der Formel (I) sich zur vorteilhaften Verwendung in Lichtsammelsystemen eignen, da zahlreiche hervorragende fluorzeszierende Farbstoffe, z.B. Rhodamin G, nicht brauchbar sind. Ebenso sind die schon sehr weitgehenden Forderungen, die an die optische Qualität von Laserfarbstoffen gestellt werden, in vielen Fällen nicht ausreichend, um die Verwendung dieser Farbstoffe in Lichtsammelsystemen zu empfehlen.

## Beispeil 1

3-(4,5-Dichlorthiazolyl-2)-4-cyano-7-diethylamino-cumarin

28 g (0,07 Mol) 3-(4,5-Dichlorthiazolyl-2)-4-chlor-7-diethylamino-cumarin werden zusammen mit 15,9 g (0,21 Mol) Kupfer(I)cyanid und 1 g Kaliumjodid in 300 ml Dimethylformamid 1 Stunde bei 100°C gerührt. Nach dem Abkühlen wird der ausgefallene Niederschlag abgesaugt, in 100 ml konz. Ammoniak ausgerührt, abgesaugt, mit konz. Ammoniak und Wasser gewaschen und getrocknet. Man erhält 27 g (97,8 %) 3-(4,5-Dichlorthiazolyl-2)-4-cyano-7-diethylamino-cumarin in Form von grün-gold schillernden Kristallen, die nach dem Umlösen aus o-Dichlorbenzol/Tonsil bei 284°C schmelzen. $\lambda_{max}$: 544,9 nm (DMF)

Analog wurden hergestellt

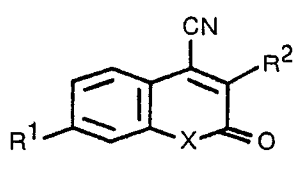

| Bsp. | $R^1$ | X | $R^2$ | |
|---|---|---|---|---|
| 2 | $N(CH_3)_2$ | O | 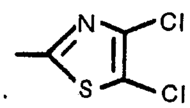 | Fp. 310°C $\lambda_{max}$: 542,4 nm (DMF) |
| 3 | „ | $N—C_2H_5$ | „ | Fp. 296°C, $\lambda_{max}$: 504,3 nm (DMF) |
| 4 | $N(C_2H_5)_2$ | $N—C_2H_5$ | „ | Fp. 247°C, $\lambda_{max}$: 508,4 nm (DMF) |
| 5 | | $N—C_2H_5$ | | 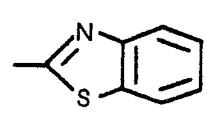 |
| | | $N(CH_3)_2$ | | |

| Bsp. | $R^1$ | X | $R^2$ | |
|---|---|---|---|---|
| 6 | $N(C_2H_5)_2$ | $N-C_2H_5$ | 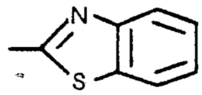 | |
| 7 | $N(C_2H_5)_2$ | O | 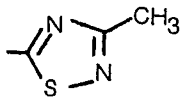 | |
| 8 | $N(C_2H_5)_2$ | O | 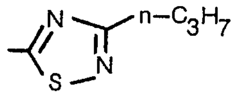 | $\lambda_{max}$: 533,3 nm (DMF) Fp. 224—6°C |
| 9 | $N(C_2H_5)_2$ | O | 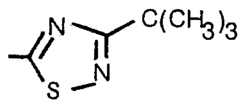 | $\lambda_{max}$: 533,3 nm (DMF) Fp. 350°C |
| 10 | $N(C_2H_5)_2$ | O | 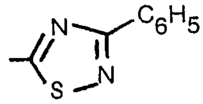 | $\lambda_{max}$: 542 nm (DMF) Fp. 265°C |

### Beispiel 11

3-(4,5-Dichlor-thiazolyl-2)-4-chlor-7-dimethylamino-cumarin

4,5 g (0,012 Mol) 3-(4,5-Dichlor-thiazolyl-2)-4-hydroxy-7-dimethylamino-cumarin werden in 150 ml o-Dichlor-benzol vorgelegt. Die Suspension wird mit gasförmigen Chlorwasserstoff gesättigt und mit 2 ml Dimethylformamid versetzt. Man erwärmt auf 100°C und leitet 4 Stunden lang einen kräftigen Phosgenstrom in die Suspension.

Es entsteht eine trübe Lösung, die nach Entfernen des überschüssigen Phosgens und Aufkochen mit A-Kohle im Vakuum eingedampft wird. Nach dem Anrühren des Rückstandes in 25 ml Methanol erhält man 4 g (84%) 3-(4,5-Dichlor-thiazolyl-2)-4-chlor-7-dimethylamino-cumarin als gelbrote Kristalle, die nach dem Umlösen aus Toluol/Tonsil bei 290°C schmelzen.

Analog werden hergestellt:

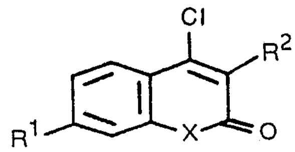

| Bsp. | $R^1$ | X | $R^2$ | |
|---|---|---|---|---|
| 12 | $N(C_2H_5)_2$ | O | 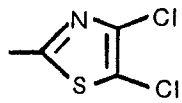 | Fp. 232—34°C |
| 13 | $N(CH_3)_2$ | $N—C_2H_5$ | " | Fp. 260—62°C |
| 14 | $N(C_2H_5)_2$ | $N—C_2H_5$ | " | |
| 15 | $N(CH_3)_2$ | O | 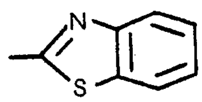 | |
| 16 | " | $N—C_2H_5$ | " | |
| 17 | $N(C_2H_5)_2$ | O | " | |
| 18 | " | $N—C_2H_5$ | " | |
| 19 | " | O | 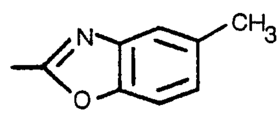 | |
| 20 | " | O | 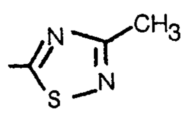 | Fp. 152—54°C |
| 20a | $N(C_2H_5)_2$ | O | 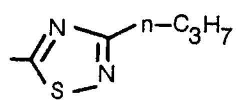 | Fp. 132—34°C |
| 20b | $N(C_2H_5)_2$ | O | 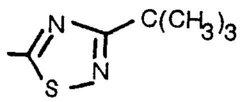 | Fp. 152—53°C |
| 20c | $N(C_2H_5)_2$ | O | 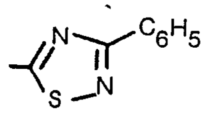 | Fp. 210°C |

Herstellung der Ausgansverbindungen IX

Beispiel 21

3-(3-Methyl-1,2,4-thiadiazolyl-5)-4-hydroxy-7-diethylamino-cumarin

98 g (0,38 Mol) (3-Methyl-1,2,4-thiadiazolyl-5)-malonsäure-diethylester und 62,7 g (0,38 Mol) 3-Diethylaminophenol werden in 400 ml o-Dichlorbenzol gelöst und erwärmt. Über eine Destillationsbrücke destilliert man den bei der Reaktion gebildeten Alkohol ab. Wenn die Sumpftemperatur 180°C erreicht hat wird noch 1 Stunde bei dieser Temperatur gerührt und anschließend auf Raumtemperatur abgekühlt. Der ausgefallene Niederschlag wird abgesaugt und mit Methanol gewaschen. Nach dem Trocknen erhält man 97 g (77%) 3-(3-Methyl-1,2,4-thiadiazolyl-5)-4-hydroxy-7-diethylamino-cumarin, die nach dem Umlösen aus Chlorbenzol/Tonsil bei 248—50°C schmelzen.

Analog werden hergestellt:

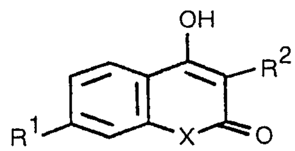

| Bsp. | $R^1$ | X | $R^2$ | |
|---|---|---|---|---|
| 22 | $N(CH_3)_2$ | O | 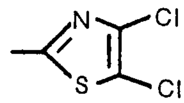 | Fp. 289°C |
| 23 | $N(CH_3)_2$ | $N—C_2H_5$ | " | Fp. 272°C |
| 24 | $N(C_2H_5)_2$ | O | " | Fp. 225°C |
| 25 | $N(C_2H_5)_2$ | $N—C_2H_5$ | " | |
| 26 | $N(CH_3)_2$ | O | 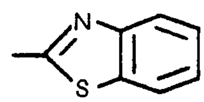 | |
| 27 | $N(CH_3)_2$ | $N—C_2H_5$ | " | |
| 28 | $N(C_2H_5)_2$ | O | " | |
| 29 | $N(C_2H_5)_2$ | $N—C_2H_5$ | " | |
| 30 | $N(C_2H_5)_2$ | O | 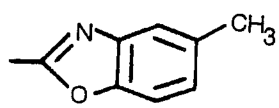 | |
| 30a | $N(C_2H_5)_2$ | O | 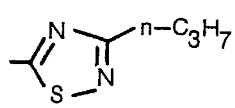 | Fp. 198°C |
| 30b | $N(C_2H_5)_2$ | O | 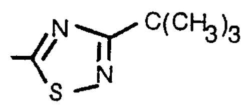 | Fp. 142—44°C |
| 30c | $N(C_2H_5)_2$ | O | 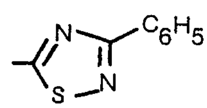 | Fp. 241°C |

Herstellung der Ausgangsverbindungen VI

Beispiel

4,5-Dichlorthiazol(2)-malonsäure-diethylester

Zu einer Mischung aus 3,77 g (0,02 Mol) Trichlorthiazol, 3,2 g (0,02 Mol) Malonsäure-diethylester und 50 g Dioxan werden bei 20°C unter Rühren 3,36 g (0,06 Mol) Kaliumhydroxid gegeben. Nach 24 Stunden wird das Festprodukt abgesaugt, mit 10 ml Dioxan nachgewaschen und getrocknet. Das getrocknete Festprodukt wird in eine Mischung aus 10 ml konzentrierter Salzsäure und 90 ml Wasser eingerührt, abgesaugt, mit Wasser gewaschen und getrocknet. Man erhält 3,9 g (62,5%) 4,5-Dichlorthiazol(2)-malonsäure-diethylester vom Schmelzpunkt 65°C.

Analog wurden erhalten:

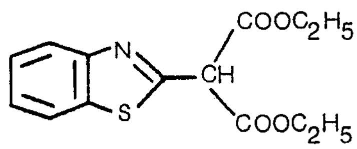

Fp: 138—9°C

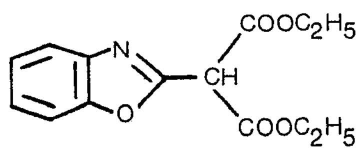

Fp: 79—80°C

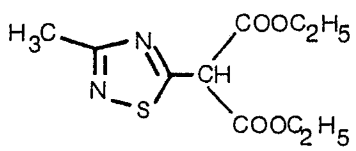

Fp: 113°C

**Patentansprüche**

1. Heterocyclische Verbindungen der Formel

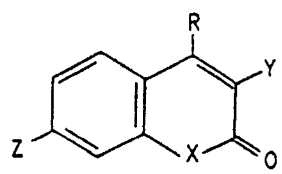

I

in der

R Cl, Br, —CN oder —OH;

X O, S oder $-NR_1$ bezeichnen, wobei

$R_1$ für Wasserstoff, $C_1$—$C_6$-Alkyl, $C_3$—$C_7$-Cycloalkyl, Phenyl, Naphthyl, Phenyl-$C_1$—$C_4$-alkyl, Naphthyl-$C_1$—$C_4$-alkyl, ($C_1$—$C_6$-Alkyl)-carbonyl, Benzoyl steht und die für $R_1$ genannten $C_1$—$C_6$-Alkyl- und ($C_1$—$C_6$-Alkyl)-carbonylreste durch —OH, —CN, $C_1$—$C_6$-Alkoxy, Phenoxy, Halogen, Amino, Carbamoyl, Sulfamoyl, ($C_1$—$C_6$-Alkyl)-carbonyloxy, ($C_1$—$C_6$-Alkoxy)-carbonyl, der $C_3$—$C_7$-Cycloalkylrest durch Halogen $C_1$—$C_6$-Alkyl, —OH, —CN, Phenyl, die Phenyl-, Naphthyl-, Benzoylreste sowie die Phenyl-$C_1$—$C_4$-alkyl und Naphthyl-$C_1$—$C_4$-alkylreste im Arylteil durch Halogen, $C_1$—$C_6$-Alkyl, $C_1$—$C_6$-Alkoxy, —CN, —OH substituiert sein können;

Y für einen 5- bis 7-gliedrigen heterocyclischen Rest, der 1, 2 oder 3 Atome aus der Reihe O, N, S enthalten kann und der anelliert sein kann, wobei die für Y genannten Reste durch Halogen, $C_1$—$C_6$-Alkyl, $C_1$—$C_6$-Alkoxy, $C_1$—$C_6$-Alkylthio, $-NO_2$, ($C_1$—$C_6$-Alkoxy)-carbonyl, Carbamoyl, Amino, Sulfamoyl substituiert sein können oder für einen 3-Phenyl-1,2,4-thiadiazol-5-yl-Rest steht;

Z Cl, Br, —CN, $R_2$—T- oder $R'_2$ bedeutet, wobei

$R_2$ für Wasserstoff, $C_1$—$C_6$-Alkyl, das durch —OH, —CN, $C_1$—$C_6$-Alkoxy, Phenoxy, Halogen, Amino, Carbamoyl, Sulfamoyl, ($C_1$—$C_6$-Alkyl)-carbonyloxy, ($C_1$—$C_6$-Alkoxy)-carbonyl substituiert sein kann, $C_3$—$C_7$-Cycloalkyl, das durch Halogen, $C_1$—$C_6$-Alkyl, —OH, —CN, Phenyl substituiert sein kann, Phenyl und Naphthyl bzw. Phenyl-$C_1$—$C_4$-alkyl und Naphthyl-$C_1$—$C_4$-alkyl, wobei die Arylreste durch Halogen, $C_1$—$C_6$-Alkyl, $C_1$—$C_6$-Alkoxy, —CN, —OH substituiert sein können, steht,

$R'_2$ für Phenyl, Naphthyl, Phenyl-$C_1$—$C_4$-alkyl, Naphthyl-$C_1$—$C_4$-alkyl, wobei die Arylreste, wie vorstehend im Zusammenhang mit $R_2$ angegeben, substituiert sein können und

T für O, S,

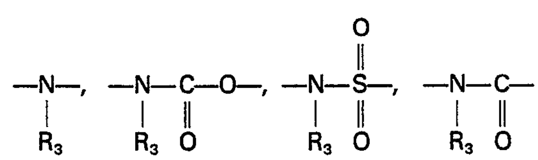

steht

$R_3$ die gleichen Bedeutungen wie $R_2$ hat, und wobei bei Vorliegen des Strukturmerkmals

$R_2$—N—,
|
$R_3$

$R_2$ und $R_3$ zusammen mit dem N-Atom einen 5- bis 7-gliedrigen Heterocyclus bilden können, mit der Maßgabe, daß

a) für den Fall, daß R —CN bezeichnet, und X für

—$NR_1$
|

steht, Z nicht $R_2$—O— bedeutet und

b) für den Fall, daß R —CN bezeichnet und X für O steht,

Y 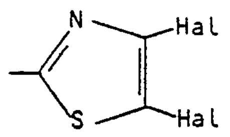 oder 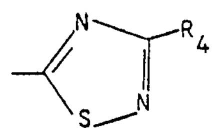

bedeutet, wobei Hal Halogen, vorzugsweise Chlor und Brom, und $R_4$ Wasserstoff, $C_1$—$C_6$-Alkyl oder Phenyl bezeichnen und

c) von diesem Anspruch die Verbindung der Formel

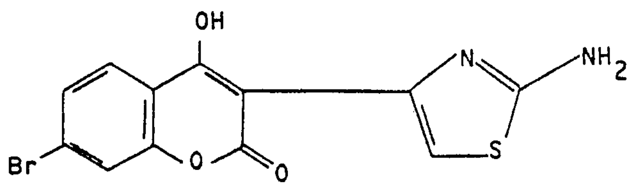

ausgenommen ist.

2. Verbindungen gemäß Anspruch 1, bei denen Y für einen Rest aus der Thiazol-, Oxazol-, 1,3,4-Oxadiazol-, 1,3,4-Thiadiazol-, 1,2,4-Thiadiazol-, Benzthiazol-, Benzoxazol-, Imidazol-, 1,2,4-Oxadiazol-, Chinazolon-, Benzimidazol-, Pyridin-, Chinolin- oder Pyrimidin-Reihe steht.

3. Verbindungen gemäß Anspruch 1, bei denen Y

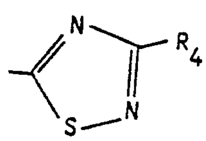

bezeichnet, insbesondere solche, bei denen R zusätzlich für CN steht.

4. Verbindungen gemäß Anspruch 1 der Formel

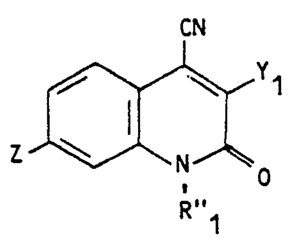

II

in der

R''$_1$ $C_1$—$C_6$-Alkyl oder Phenyl und

$Y_1$ einen gegebenenfalls durch Chlor, Brom, oder $C_1$—$C_4$-Alkyl insbesondere Methyl substituierten Benzthiazolyl(2)-, Benzoxazolyl(2)-, Thiazolyl(2)-, 1,2,4-Thiadiazolyl(5)-, 1,3,4-Thiadiazolyl(2)- oder 1,3,4-Oxadiazolyl(2)-Rest bezeichnen und

Z die in Anspruch 1 angegebene Bedeutung hat.

5. Verbindungen der Formel

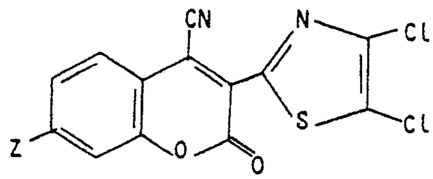

III

in der Z die in Anspruch 1 angegebene Bedeutung hat.

6. Verbindungen der Formel

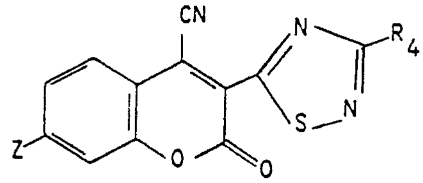

in der

$R_4$ Wasserstoff, $C_1$—$C_6$-Alkyl oder Phenyl bezeichnet und

Z die in Anspruch 1 angegebene Bedeutung hat.

7. Verbindungen gamäß Anspruch 1, bei denen R Cl bedeutet.

8. Verwendung von Verbindungen gemäß den Ansprüchen 1—7 zum Färben oder Bedrucken von synthetischen oder halbsynthetischen Materialien.

9. Verwendung der Verbindungen gemäß den Ansprüchen 1—6, insbesondere von solchen bei denen R für Cyan steht, als Lichtwandler in Lichtsammelsystemen.

**Revendications**

1. Composés hétérocycliques de formule:

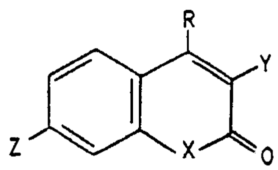

I

dans laquelle

R représente Cl, Br, —CN ou —OH;

X représente O, S ou —NR$_1$ dans lequel
|

$R_1$ représente l'hydrogène ou un groupe alkyle en $C_1$—$C_6$, cycloalkyle en $C_3$—$C_7$, phényle, naphtyle, phényl-alkyle en $C_1$—$C_4$, naphtylalkyle en $C_1$—$C_4$, (alkyl en $C_1$—$C_6$)-carbonyle ou benzoyle et les restes alkyles en $C_1$—$C_6$ et (alkyl en $C_1$—$C_6$)-carbonyles cités pour $R_1$ peuvent être substitués par —OH, —CN, alcoxy en $C_1$—$C_6$, phénoxy, halogène, amino, carbamoyle, sulfamoyle, (alkyl en $C_1$—$C_6$)-carbonyloxy, (alcoxy en $C_1$—$C_6$)-carbonyle, le reste cycloalkyle en $C_3$—$C_7$ par halogène, alkyle en $C_1$—$C_6$, —OH, —CN, phényle, les restes phényle, naphtyle, benzoyle ainsi que les restes phényl-alkyles en $C_1$—$C_4$ et naphtyl-alkyles en $C_1$—$C_4$ peuvent être substitués au noyau par halogène, alkyle en $C_1$—$C_6$, alcoxy en $C_1$—$C_6$, —CN, —OH;

Y représente un reste hétérocyclique ayant 5 à 7 chaînons, qui peut contenir 1, 2 ou 3 atomes de la série O, N, S et qui peut être condensé, les restes cités pour Y pouvant être substitués par halogène, alkyle en $C_1$—$C_6$, alcoxy en $C_1$—$C_6$, alkylthio en $C_1$—$C_6$, —$NO_2$, (alcoxy en $C_1$—$C_6$)-carbonyle, carbamoyle, amino ou sulfamoyle, ou Y est un reste 3-phényl-1,2,4-thiadiazole-5-yle;

Z représente Cl, Br, —CN, $R_2$—T— ou $R'_2$,

$R_2$ représente l'hydrogène, un groupe alkyle en $C_1$—$C_6$ qui peut être substitué par —OH, —CN, alcoxy en $C_1$—$C_6$, phénoxy, halogène, amino, carbamoyle, sulfamoyle, (alkyl en $C_1$—$C_6$)-carbonyloxy, (alcoxy en $C_1$—$C_6$)-carbonyle, un groupe cycloalkyle en $C_3$—$C_7$ qui peut être substitué par halogène, alkyle en $C_1$—$C_6$, —OH, —CN, phényle, un groupe phényle ou naphtyle ou phényl-alkyle en $C_1$—$C_4$ ou naphtyl-alkyle en $C_1$—$C_4$, les restes aryles pouvant être substitués par halogène, alkyle en $C_1$—$C_6$, alcoxy en $C_1$—$C_6$, —CN, —OH,

$R'_2$ représente un groupe phényle, naphtyle, phényl-alkyle en $C_1$—$C_4$, naphtyl-alkyle en $C_1$—$C_4$, les restes aryles pouvant être substitués comme indiqué précédemment pour $R_2$ et

T représente O, S, 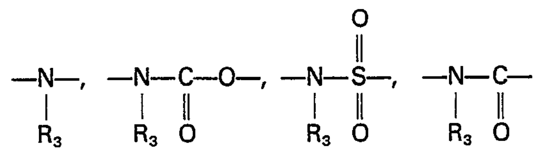

$R_3$ a les mêmes significations que $R_2$ et, dans le cas de la présence de l'élément de structure

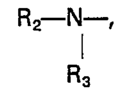

$R_2$ et $R_3$ pris ensemble avec l'atome d'azote peuvent former un hétérocycle à 5—7 chaînons, avec la condition que

a) dans le cas où R représente —CN et X représente

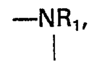

le reste Z ne représente pas $R_2$—O— et

b) dans le cas où R représente —CN et X représente O,

Y représente

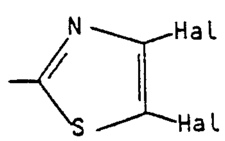

ou

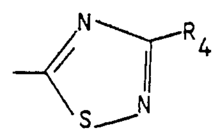

dans lesquels Hal représente un halogène, de préférence le chlore et le brome, et $R_4$ représente l'hydrogène, alkyle en $C_1$—$C_6$ ou phényle et

c) le composé de formule

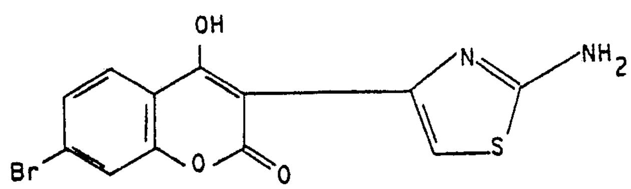

est exclus de cette revendication.

2. Composés selon la revendication 1, dans lesquels

Y représente un reste de la série du thiazole, de l'oxazole, du 1,3,4-oxadiazole, du 1,3,4-thiadiazole, du 1,2,4-thiadiazole, du benzothiazole, du benzoxazole, de l'imidazole, du 1,2,4-oxodiazole, de la quinazolone, du benzimidazole, de la pyridine, de la quinoléine ou de la pyrimidine.

3. Composés selon la revendication 1, dans lesquels

Y représente

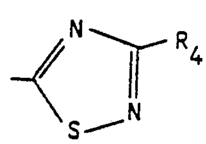

en particulier ceux dans lesquels R représente en outre CN.

4. Composés selon la revendication 1, de formule

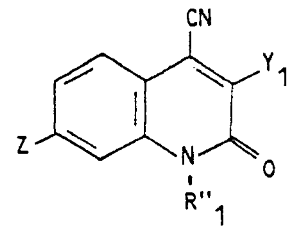

II

dans laquelle

$R''_1$ représente un groupe alkyle en $C_1$—$C_6$ ou phényle et

$Y_1$ représente un reste benzothiazolyl(2), benzoxazolyle(2), thiazolyle(2), 1,2,4-thiadiazolyle(5), 1,3,4-thiadiazolyle(2) ou 1,3,4-oxadiazolyle(2), éventuellement substitué par le chlore, le brome ou un groupe alkyle en $C_1$—$C_4$, en particulier méthyle, et

Z a la signification indiquée à la revendication 1.

5. Composés de formule:

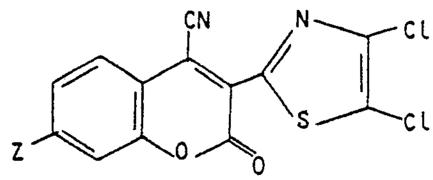

III

dans laquelle Z a la signification indiquée à la revendication 1.

6. Composés de formule

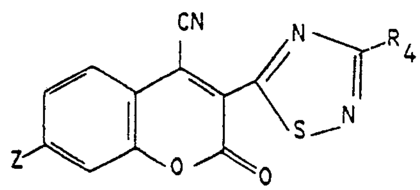

dans laquelle

$R_4$ représente l'hydrogène ou un groupe alkyle en $C_1$—$C_6$ ou phényle et

Z a la signification indiquée à la revendication 1.

7. Composés selon la revendication 1, dans lequels R représente Cl.

8. Utilisation de composés selon les revendications 1 à 7, pour la teinture ou l'impression de matières synthétiques ou semi-synthétiques.

9. Utilisation des composés selon les revendications 1 à 6, en particulier de ceux dans lesquels R représente un groupe cyano, comme convertisseurs de lumière dans des systèmes collecteurs de lumière.

**Claims**

1. Heterocyclic compounds corresponding to the following formula

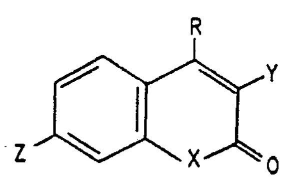

I

in which

R is Cl, Br, —CN or —OH;

X is O, S or —NR$_1$ where

$R_1$ is hydrogen, $C_1$—$C_6$ alkyl, $C_3$—$C_7$ cycloalkyl, phenyl, naphthyl, phenyl-$C_1$—$C_4$-alkyl, naphthyl-C—$C_4$-alkyl, ($C_1$—$C_6$-alkyl)-carbonyl, benzoyl and the $C_1$—$C_6$ alkyl radicals and ($C_1$—$C_6$-alkyl)-carbonyl radicals mentioned for $R_1$ may be substituted by —OH, —CN, $C_1$—$C_6$ alkoxy, phenoxy, halogen, amino, carbamoyl, sulfamoyl, ($C_1$—$C_6$-alkyl)-carbonyloxy, ($C_1$—$C_6$-alkoxy)-carbonyl, the $C_3$—$C_7$ cycloalkyl radical by halogen, $C_1$—$C_6$ alkyl, —OH, —CN, phenyl, the phenyl, naphthyl, benzoyl radicals and the phenyl-$C_1$—$C_4$-alkyl and naphthyl-$C_1$—$C_4$-alkyl radicals in the aryl part by halogen, $C_1$—$C_6$ alkyl, $C_1$—$C_6$ alkoxy, —CN, —OH;

Y is a 5- to 7-membered heterocyclic radical which may contain 1, 2 or 3 atoms from series O, N, S and which may be fused, the radicals mentioned for Y optionally being substituted by halogen, $C_1$—$C_6$ alkyl, $C_1$—$C_6$ alkoxy, $C_1$—$C_6$ alkylthio, —$NO_2$, ($C_1$—$C_6$-alkoxy)-carbonyl, carbamoyl, amino, sulfamoyl or representing a 3-phenyl-1,2,4-thiadiazol-5-yl radical;

Z is Cl, Br, —CN, $R_2$,T or $R'_2$ where

$R_2$ is hydrogen, $C_1$—$C_6$ alkyl which may be substituted by —OH, —CN, $C_1$—$C_6$ alkoxy, phenoxy, halogen, amino, carbamoyl, sulfamoyl, ($C_1$—$C_6$-alkyl)-carbonyloxy, ($C_1$—$C_6$-alkoxy)-carbonyl, $C_3$—$C_7$ cycloalkyl which may be substituted by halogen, $C_1$—$C_6$ alkyl, —OH, —CN, phenyl; phenyl and naphthyl or phenyl-$C_1$—$C_4$-alkyl and naphthyl-$C_1$—$C_4$-alkyl, the aryl radicals optionally being substituted by halogen, $C_1$—$C_6$ alkyl, $C_1$—$C_6$ alkoxy, —CN, —OH,

$R'_2$ is phenyl, naphthyl, phenyl-$C_1$—$C_4$-alkyl, naphthyl-$C_1$—$C_4$-alkyl, the aryl radicals optionally being substituted as indicated above in connection with $R_2$ and

T represents O, S, 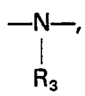 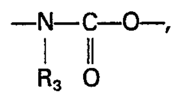 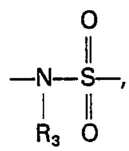 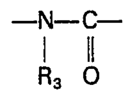

$R_3$ has the same meanings as $R_2$ and, where the structural feature

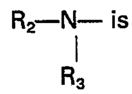 is present,

$R_2$ and $R_3$ together with the N atom may form a 5- to 7-membered ring, with the proviso that

a) where R is —CN and X is

—$NR_1$

Z is not $R_2$—O— and

b) where R is —CN and X is O, Y represents

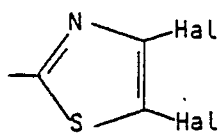

or

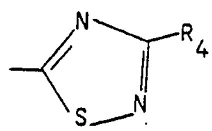

where Hal is halogen, preferably chlorine and bromine, and $R_4$ is hydrogen, $C_1$—$C_6$ alkyl or phenyl and

c) the compound

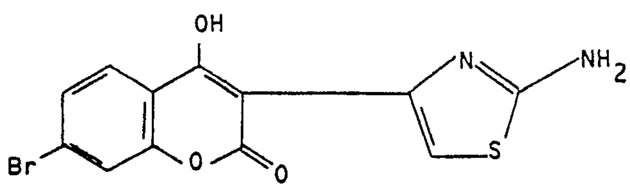

is excepted from this claim.

2. Compounds as claimed in Claim 1 in which

Y is a radical from the thiazole, oxazole, 1,3,4-oxadiazole, 1,3,4-thiadiazole, 1,2,4-thiadiazole, benzthiazole, benzoxazole, imidazole, 1,2,4-oxadiazole, quinazolone, benzimidazole, pyridine, quinole or pyrimidine series.

3. Compounds as claimed in Claim 1 in which Y represents

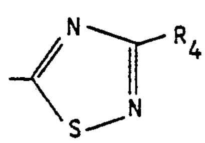

more especially those in which R is additionally CN.

4. Compounds as claimed in Claim 1 corresponding to the following formula

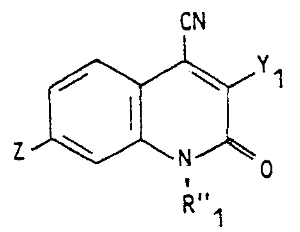

II

in which

$R''_1$ is $C_1$—$C_6$ alkyl or phenyl and

$Y_1$ is a benzthiazol-2-yl, benzoxazol-2-yl, thiazol-2-yl, 1,2,4-thiadiazol-5-yl, 1,3,4-thiadiazol-2-yl or 1,3,4-oxadiazol-2-yl radical optionally substituted by chlorine, bromine or $C_1$—$C_4$ alkyl, more especially methyl, and

Z is as defined in Claim 1.

5. Compounds corresponding to the following formula

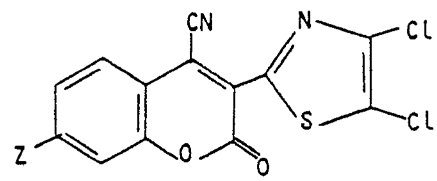

III

in which Z is as defined in Claim 1.

6. Compounds corresponding to the following formula

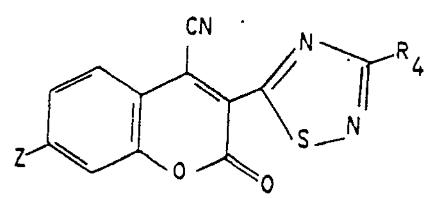

in which

$R_4$ is hydrogen, $C_1$—$C_6$ alkyl or phenyl and

Z is as defined in Claim 1.

7. Compounds as claimed in Claim 1 in which R is Cl.

8. The use of the compounds claimed in claims 1 to 7 for dyeing or printing synthetic or semisynthetic materials.

9. The use of the compounds claimed in Claims 1 to 6, more especially those in which R is cyano, as phototransducers in light collecting systems.